# EUROPEAN PATENT APPLICATION

(11) **EP 1 262 203 A2**
(43) Date of publication of application: **04.12.2002**
(21) Application number: 02253776.5
(22) Date of filing: 29.05.2002
(51) Int. Cl.: A61M 1/14

(54) **Portable hemodialyzer**

(30) Priority: 29.05.2001 KR 2001029608
(71) Applicant: Kim, Kyoung Jin, Yonsoo-gu, Incheon-shi (KR)
(72) Inventor: Kim, Kyoung Jin, Yonsoo-gu, Incheon-shi (KR)
(74) Representative: Brown, Kenneth Richard

(57) **Abstract**

Disclosed is a portable hemodialyzer, which not only can relieve a patient from the pain of putting injection needles into two portions of the patient's body for hemodialysis, but also enables the hemodialysis to be safely performed even at home, so as to reduce the times by which the patient must visit the hospital, thereby largely reducing time and expense. In the portable hemodialyzer, an injection needle (10) contains a separation film (15) separating an introduction channel (13) and a discharge channel (14) from each other, which are formed in the injection needle. A blood exit (12) is formed at a predetermined left side portion of the injection needle, while a blood entrance (11) is formed at a predetermined right side portion of the injection needle. Therefore, the blood introduced through the blood entrance (11) by the pumping operation by the driving force of the driving section is delivered through the introduction channel (13) to the hemodialysis filter, filtered by the hemodialysis filter, and then supplied through the discharge channel (14) and the blood exit (12) to the blood vessel.

## Description

### BACKGROUND OF THE INVENTION

### Field of the invention

The present invention relates to a hemodialyzer, and more particularly to a portable hemodialyzer, which not only can relieve a patient from the pain of putting injection needles into two portions of the patient's body for hemodialysis, but also enables the hemodialysis to be safely performed even at home, so as to reduce the times by which the patient must visit the hospital, thereby largely reducing time and expense.

### Description of the Prior Art

As generally known in the art, blood means a liquid system flowing in a blood vessel. Blood has a clear red color, is opaque, and looks homogeneous. However, when a small drop of blood is spread thinly on a glass plate and observed through a microscope, it is noticed that blood-corpuscles float in transparent liquid, which means the blood is not homogeneous. A main function of the blood is to carry various materials. That is, the blood delivers oxygen absorbed through the lungs and nutritive substance absorbed through the digestive canal to all cells of the body, and carries and discharges carbonic acid gas or waste materials, produced in the cells, out of the body through the lungs, kidneys, or skin. Further, the blood transfers heat from portions such as the skeletal muscles and liver, in which heat is vigorously produced, to other portions, thereby equalizing the distribution of the body heat. Moreover, the blood helps the skin to discharge heat, thereby maintaining the body temperature constant.

The blood consists of hematocyte, leucocyte, thrombocyte, and blood plasma. The hematocyte is shaped like a disc and has no nucleus. The leucocyte has no specific shape and a nucleus. The thrombocyte is relatively small and has no nucleus. The blood plasma is the liquid component of the blood. The hematocyte takes 45% of the entire blood and performs the function of carrying the oxygen. Further, the leucocyte performs phagocytosis, the thrombocyte relates to coagulation of the blood, and the blood plasma relates to the transfer of materials. The leucocyte, which is largest among said components of the blood, has no regular shape and a size between 12 and 25 µm, and the hematocyte has a diameter of 8 µm and is shaped like a disc, central portions of which are concave.

According to the definition in Chinese medical science, extravasated blood is a disease caused by increase of waste material in a certain portion of the body due to stagnation of blood in the portion. Blood which is dead after leaking out of the blood vessel and then being collected in a gap of tissue is defined as bad blood, and blood staying in organs or blood vessels due to deterioration of the blood circulation is defined as accumulated blood. The bad blood and the accumulated blood also belong to the extravasated blood. In more detailed description, when food is changed to waste material after being digested, the waste material is excreted out of the body by various organs. In this case, when the organs have trouble or cannot make the waste material be smoothly excreted due to aging, etc., the waste material stays in blood or lymph, thereby causing disease. The blood containing too much waste material as described above is the extravasated blood.

When the extravasated blood stagnates in blood vessel or tissue, there appears a symptom in which a patient's mouth dries. Further, the patient may feel his or her abdomen inflating and all portions of his or her body hot. Moreover, purple spot or blue spot may appear on skin or mucous membrane, the skin may become rough like shark's skin, and stool may be black and is apt to contain blood. In other words, the stagnation of the extravasated blood may cause various diseases dealt in dermatology, ophthalmology, neurology, obstetrics and genecology, or internal treatment.

In the meantime, hemodialysis is means generally used in treating acute and chronic renal failure. In the hemodialysis, at first, a solution capable of eliminating nitrogen-containing waste material, which is produced due to too much metabolism of water and protein, from the patient's blood, maintaining balance between acid and base in the blood plasma, and improving concentration of electrolyte in the blood plasma, is prepared. Then, a tube made of a dialysis film is put in the solution, and a portion of the patient's blood is circulated through the tube, so that the principal function of the patient's kidney is carried out by this apparatus. This apparatus performing the hemodialysis as described above is called an artificial kidney.

That is to say, the hemodialysis is a process of eliminating waste material and water from the blood by means of the artificial kidney. The artificial kidney contains a film made from simple material and having channels or holes which are very small in such a degree that they are visible only through a microscope. In this case, particles smaller than the channels or holes can pass but particles larger than the channels or holes cannot pass through the film. In order to perform the hemodialysis, in which the waste material and water are eliminated by a semipermeable membrane as described above, a hemodialyzer and an artificial kidney are necessary. When the hemodialysis is carried out, intravenous injection must be given to two portions of the patient's arm.

In a room for the hemodialysis, the patient lies on a bed and two needles for the intravenous injection are pricked into two portions of the patient's arm. In this case, the patient's blood is drawn out of the patient's body through one injection needle, while the blood after passing through the hemodialyzer is injected into the patient's body through the other injection needle. In the artificial kidney, when the blood flows through the interior of the semipermeable membrane, diffusate flows outside of the semipermeable membrane, so that the hemodialysis is carried out. The hemodialysis is a process of eliminating water and small particles such as electrolytes and waste materials, which cannot be discharged through the kidney, by means of the principle of diffusion and hydrostatic pressure.

In order to perform the hemodialysis, the blood must be drawn out of and injected into the patient's body at a rate of 200 ml per minute. In this case, since it is difficult to draw sufficient blood out of the patient's body through peripheral blood vessels, an operation for connecting an artery and a vein with each other is necessary. This operation is carried out in an operation room by means of local anesthesia, and a portion located one or two inches over a wrist is usually operated. After the blood vessel is operated, arterial blood flows through the vein, so that the vein is enlarged and becomes strong. Therefore, when the patient touches the operated portion, he or she feels tingling or buzzing. Further, when stethoscope is applied to the operated portion, the buzzing sounds like a thunder through the stethoscope. It can be said that the operation was done well when there exist such feeling and sound. The hemodialysis can be performed after passing at least one month (two month in the case of a diabetic) from the operation.

For the hemodialysis, the patient must periodically visit the hospital. Therefore, the patient can take periodical medical examination from doctors, and can do normal social activities by receiving the medical treatment two or three times each week. However, the patient cannot avoid the trouble of visiting the hospital according to a fixed schedule and the pain of getting pricked two times by the injection needles whenever he or she receive the medical treatment. Moreover, the patient must undergo dietary treatment and ingest water under certain restriction, in addition to the medical treatment. Furthermore, since the patient must visit the hospital whenever he or she receives the medical treatment, he or she must expend too much medical cost.

### SUMMARY OF THE INVENTION

Accordingly, the present invention has been made to solve the above-mentioned problems occurring in the prior art, and an object of the present invention is to provide a portable hemodialyzer, which not only can relieve a patient from the pain of putting injection needles into two portions of the patient's body for hemodialysis, but also enables the hemodialysis to be safely performed even at home, so as to reduce the times by which the patient must visit the hospital, thereby largely reducing time and expense.

In order to accomplish this object, there is provided a portable hemodialyzer comprising: an injection needle containing a separation film separating an introduction channel and a discharge channel from each other, which are formed in the injection needle; medical tubes connected with the injection needle, so as to transport blood; a hemodialysis filter filtering the blood supplied through the medical tubes, the blood being discharged from the hemodialysis filter after being filtered; a sensor section disposed at one side portion of the hemodialysis filter and generating predetermined electric signals according to flowing velocities of the blood; a driving section generating driving force which enables the blood to be hemodialyzed by the hemodialysis filter; and a control section controlling the driving section, the control section stopping the driving section when the sensor section generates a predetermined electric signal.

In the portable hemodialyzer according to the present invention as described above, the blood introduced through the blood entrance by the pumping operation by the driving force of the driving section is delivered through the introduction channel to the hemodialysis filter, filtered by the hemodialysis filter, and then supplied through the discharge channel and the blood exit to the blood vessel.

### BRIEF DESCRIPTION OF THE DRAWINGS

The above and other objects, features and advantages of the present invention will be more apparent from the following detailed description taken in conjunction with the accompanying drawings, in which:
FIG. 1 is an enlarged sectional view of an injection needle employed in a portable hemodialyzer according to the present invention;
FIG. 2 is a schematic side view showing a state that the injection needle is put in a blood vessel; and
FIG. 3 is a block diagram of a portable hemodialyzer according to the present invention.

### DETAILED DESCRIPTION OF THE PREFERRED EMBODIMENTS

Hereinafter, a portable hemodialyzer according to a preferred embodiment of the present invention will be described in detail with reference to the accompanying drawings.

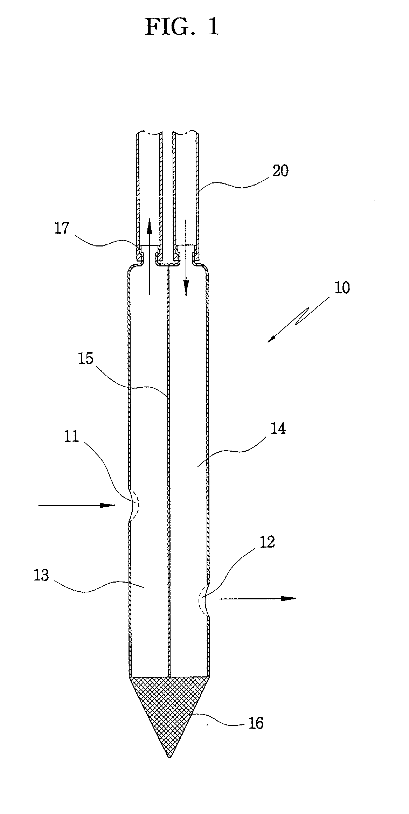

Referring to FIG. 1, an injection needle 10 employed in a portable hemodialyzer according to the present invention contains a separation film 15 separating a discharge channel 14 and an introduction channel 13 from each other, which are formed in the injection needle 10. A needle end 16 has a pointed shape which enables the injection needle 10 to be easily put into a blood vessel. The needle end 16 is closed so that the blood cannot flow into and out of the injection needle 10 through the needle end 16. Further, a blood exit 12 is formed at a predetermined portion of the discharge channel 14 of the injection needle 10 so that the blood can be discharged through the blood exit 12 out of the injection needle 10. A blood entrance 11 is formed at a predetermined portion of the introduction channel 13 of the injection needle 10 so that the blood can be introduced through the blood entrance 11 into the injection needle 10.

Meanwhile, the blood exit 12 is formed adjacent to the needle end 16, while the blood entrance 11 is spaced a predetermined distance from the needle end 16, so that the blood discharged through the blood exit 12 is prevented from being introduced again through the blood entrance 11.

Further, a connecting portion 17 is formed at an upper end of the injection needle 10, so that two medical tubes 20 can be detachably assembled with the connecting portion 17. The connecting portion 17 has jaws preventing the medical tubes 20 from being easily separated from the connecting portion 17.

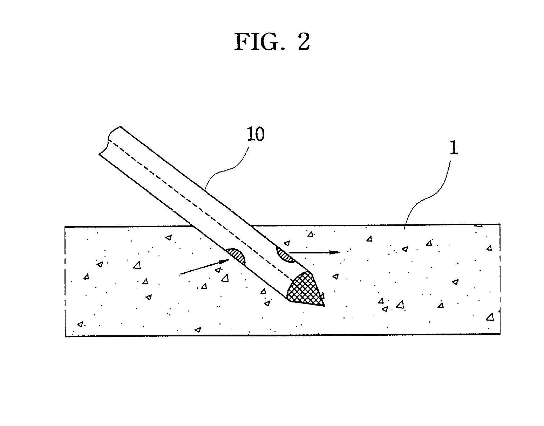

That is, as shown in FIG. 2, when the injection needle 10 is put into a blood vessel 1 of a human body while being inclined, the blood is introduced through the blood entrance 11 into the injection needle 10 while being discharged through the blood exit 12 out of the injection needle 10, thereby preventing the injection needle 10 from disturbing flow of the blood in the blood vessel the blood vessel 1.

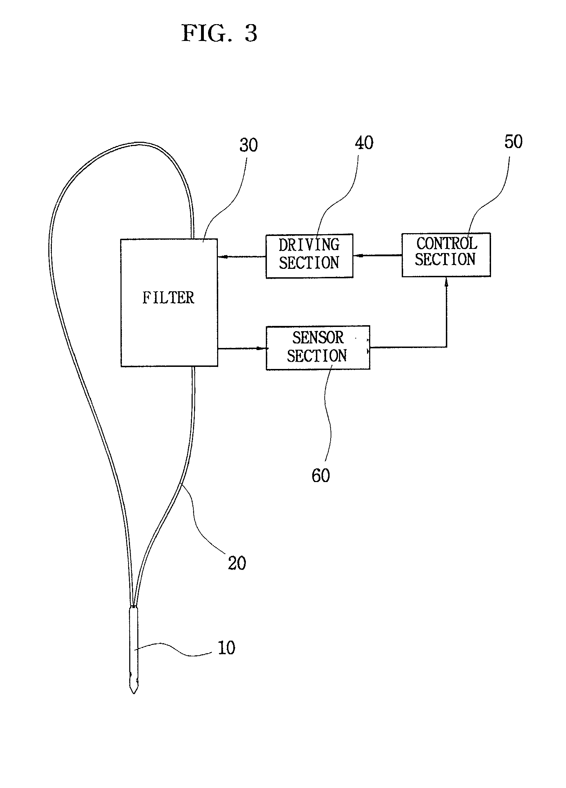

As shown in FIG. 3, the medical tubes 20 are connected with upper and lower ends of a hemodialysis filter 30. The blood introduced through the blood entrance 11 and the introduction channel 13 of the injection needle 10 is supplied to the upper end of the hemodialysis filter 30 by driving force of a driving section 40, filtered by the hemodialysis filter 30, and then discharged out of the hemodialysis filter 30 through the lower end of the hemodialysis filter 30. Then, the clean blood discharged through the lower end of the hemodialysis filter 30 is supplied through the medical tube 20, and the discharge channel 14 and the blood exit 12 of the injection needle 10 to the blood vessel 1.

A hollow textile filter which is widely used may be employed as the hemodialysis filter 30, and heparin is added to the hemodialysis filter 30 so as to prevent the blood from coagulated in the hemodialysis filter 30. Further, a sensor section 60 is disposed at a predetermined side portion of the hemodialysis filter 30 and includes a blood flow sensor (not shown) for measuring velocity of the blood flow. The sensor section 60 generates predetermined electric signals according to the velocity of the blood flow discharged after being filtered, by means of the principle of a general flow meter.

The electric signal of the sensor section 60 is transmitted to a control section 50, and the control section 50 generates a control signal to stop the driving section 40 when the velocity of the blood flow is larger than a preset velocity of the blood flow. The velocity of the blood flow preset in the control section 50 can be adjusted according to blood vessels subjected to the hemodialysis, in consideration of predetermined errors in advance, thereby preventing the blood vessel and human body from being overstrained.

As a motor (not shown) of the driving section 40, an alternating current motor may be employed so that the hemodialyzer can be used at home. Otherwise, a direct current (DC) motor may also be employed according to the necessity. The driving force of the motor operates a pump (not shown) provided at the driving section 40, and the hemodialysis filter 30 can perform the filtering operation by pumping operation of the pump.

In a portable hemodialyzer according to the present invention as described above, the injection needle 10 contains the separation film 15 separating the introduction channel 13 and the discharge channel 14 from each other, which are formed in the injection needle 10. The blood exit 12 is formed at a predetermined left side portion of the injection needle 10, while the blood entrance 11 is formed at a predetermined right side portion of the injection needle 10. Therefore, the blood introduced through the blood entrance 11 by the pumping operation by the driving force of the driving section 40 is delivered through the introduction channel 13 to the hemodialysis filter 30, filtered by the hemodialysis filter 30, and then supplied through the discharge channel 14 and the blood exit 12 to the blood vessel 1. Therefore, the portable hemodialyzer according to the present invention not only can relieve a patient from the pain of putting the injection needles into two portions of the patient's body for hemodialysis, but also enables the hemodialysis to be safely performed even at home, so as to reduce the times by which the patient must visit the hospital, thereby largely reducing time and expense.

Although a preferred embodiment of the present invention has been described for illustrative purposes, those skilled in the art will appreciate that various modifications, additions and substitutions are possible, without departing from the scope and spirit of the invention as disclosed in the accompanying claims.

## Claims

1. A portable hemodialyzer comprising:
an injection needle containing a separation film separating an introduction channel and a discharge channel from each other, which are formed in the injection needle;
medical tubes connected with the injection needle, so as to transport blood;
a hemodialysis filter filtering the blood supplied through the medical tubes, the blood being discharged from the hemodialysis filter after being filtered;
a sensor section disposed at one side portion of the hemodialysis filter and generating predetermined electric signals according to flowing velocities of the blood;
a driving section generating driving force which enables the blood to be hemodialyzed by the hemodialysis filter; and
a control section controlling the driving section, the control section stopping the driving section when the sensor section generates a predetermined electric signal.

2. A portable hemodialyzer as claimed in claim 1, wherein the separation film in the injection needle separates the introduction channel and the discharge channel from each other, and the injection needle has a blood entrance formed at a predetermined portion of the introduction channel, a blood exit formed at a predetermined portion of the discharge channel, a needle end formed at a lower end of the injection needle, and a connecting portion formed at an upper end of the injection needle, the blood being introduced through the blood entrance into the injection needle and discharged through the blood exit out of the injection needle, the needle end having a pointed shape which enables the injection needle to be easily put into a blood vessel, the needle end being closed so as to prevent the blood from flowing into and out of the injection needle through the needle end, the connecting portion having jaws which prevent the medical tubes from being easily separated from the connecting portion when the medical tubes are detachably assembled with the connecting portion.

3. An injection needle for a portable hemodialyzer, said injection needle having therein separated introduction and discharge channels, a blood entrance for passing a blood flow into the introduction channel from an injected blood vessel, and a blood exit for returning the blood flow to said blood vessel from the discharge channel.

4. An injection needle according to claim 2, wherein said introduction and exit channels extend axially of the needle with an axially extending channel separation element therebetween.

5. An injection needle according to claim 4, wherein said blood entrance and exit comprise openings formed in the outer needle wall at positions spaced axially from the needle tip.

6. An injection needle according to claim 5, wherein said openings are formed at respective positions with different axial spacings from said needle tip.

7. An injection needle according to any of claims 4 to 6, wherein the needle is provided with a connecting portion for the detachable connection of tubes to convey blood both away from the introduction channel and back into the discharge channel.
